# EUROPEAN PATENT SPECIFICATION

(11) **EP 2 486 947 B1**
(45) Date of publication and mention of the grant of the patent: **17.02.2016**
(21) Application number: 09850242.0
(22) Date of filing: 07.10.2009
(51) Int. Cl.: A61L 15/28, A61L 15/64, A61L 17/06

(54) **DEGRADABLE COMPOSITE MATERIAL CONTAINING CHITIN OR CHITOSAN**
ABBAUBARER VERBUNDSTOFF MIT CHITIN ODER CHITOSAN
MATÉRIAU COMPOSITE DÉGRADABLE CONTENANT UNE CHITINE OU UN CHITOSANE

(43) Date of publication of application: 15.08.2012
(73) Proprietor: Thermostable Enzyme Laboratory Co. Ltd., Hyogo 650-0047 (JP); Totai Co., Ltd., Taito-ku Tokyo 110-0005 (JP)
(72) Inventor: OKU, Takashi, Kobe-shi Hyogo 650-0047 (JP); NOGUCHI, Takeshi, Yufu-shi Oita 879-5103 (JP)
(74) Representative: Webster, Jeremy Mark
(86) International application number: PCT/JP2009/067522
(87) International publication number: WO 2011/042971

(56) References cited:
- WO-A1-01/45761
- GB-A- 2 150 833
- JP-A- 6 322 181
- JP-A- 10 259 347
- JP-A- H06 322 181
- JP-A- 2005 027 572

## Description

### Technical Field

The present invention relates to a degradable composite material containing at least one type of polysaccharide selected from chitin and chitosan. More specifically, the present invention relates to a degradable composite material that contains at least one type of polysaccharide selected from chitin and chitosan and that slowly degrades in an environment where moisture is present, such as a living body.

### Background Art

Chitin and chitosan are used in a variety of medical materials because they do not adversely affect ecological systems (see Patent Document 1 and 2). For example, sheets manufactured having chitin and chitosan as a raw material are used as a wound dressing material, and fibers manufactured having chitin and chitosan as a raw material are used as a medical prosthetic material. It is known that when chitin and chitosan degrade, they become soluble saccharides that are absorbed *in vivo,* and it has been learned that the degradation products thereof are highly biocompatible. There are problems, however, because chitin and chitosan cannot be broken down by enzymes in the human body, so medical materials manufactured with these substances as a raw material are difficult to use in a mode that remains in the body. Medical materials to be used such that they remain in body tissues for long periods of time have the advantage of safety and ease of use because removal of the medical material that has served its purpose becomes unnecessary if it is absorbed in the body and disappears at a stage wherein damage to the tissue has healed to a certain extent after surgery.

Therefore, medical materials with even greater utility can be provided, if a self-degrading capability can be imparted to chitin and chitosan. Until now, however, no technology capable of providing chitin and chitosan with this self-degrading capability has been established.

### Citation List

### Patent Literature

Patent Document 1: Japanese Patent Application Laid-open No. 2003-265591
Patent Document 2: Japanese Patent Application Laid-open No. 05135039 (publication no. JP 06322181)

### Summary of the Invention

### Technical Problem

An object of the present invention is to provide a degradable composite material that contains at least one type of polysaccharide selected from chitin and chitosan, and has self-degrading capability.

### Solution to Problem

The inventors conducted intensive research to solve the above problems, and they discovered that by supporting an enzyme capable of hydrolyzing chitin and chitosan on a molded article containing at least one type of polysaccharide selected therefrom, the polysaccharide can be slowly degraded in an environment where moisture is present such as a living body, and that a degradable composite material having self-degrading capability can be provided thereby. In particular, the inventors discovered that by using a thermostable enzyme as the above degrading enzyme, the degrading activity thereof on the polysaccharide can be stably maintained over a long period of time, and both sustained and safe degradation of the composite material becomes possible in an environment where moisture is present. The present invention was completed by further modifications and improvements based on this knowledge.

More specifically, the present invention provides a degradable composite material as set out in claim 1.

Also provided are the use of the degradable composite material in the manufacture of a medical material; the degradable composite material in the form of a sheet, for use in a method of treating a wound by covering the wound site with the degradable composite material; and the degradable composite material in the form of a fiber, for use in a method of suturing an incision site or a laceration site by suturing the incision site or laceration site with the degradable composite material.

### Advantageous Effects of Invention

In an environment where moisture is present the degradable composite material of chitin and/or chitosan of the present invention can self-degrade into highly biocompatible soluble saccharides. As a result, the burden on the patient can be reduced because when the degradable composite material of the present invention is applied to injured tissue as a medical material, it disappears after remaining on the injured tissue for a desired period of time, and therefore removal of the medical material after surgery becomes unnecessary.

Moreover, by using a degrading enzyme that exhibits activity over a wide temperature range of 4°C to 100°C as the degrading enzyme that can hydrolyze the chitin and/or chitosan in the degradable composite material of the present invention, self-degradation can be induced in an environment where moisture is present under a variety of temperature conditions, that this enables the present invention to be used in a broad range of applications.

In addition, by using a thermostable enzyme as the degrading enzyme in the degradable composite material of the present invention, the self-degradation capability of the degradable composite material can be stably maintained because of the high level of stability of the enzyme, and therefore sustained and stable decomposition of the degradable composite material becomes possible in an environment where moisture is present. In addition, this feature provides long-term storage stability.

### Brief Description of the Drawings

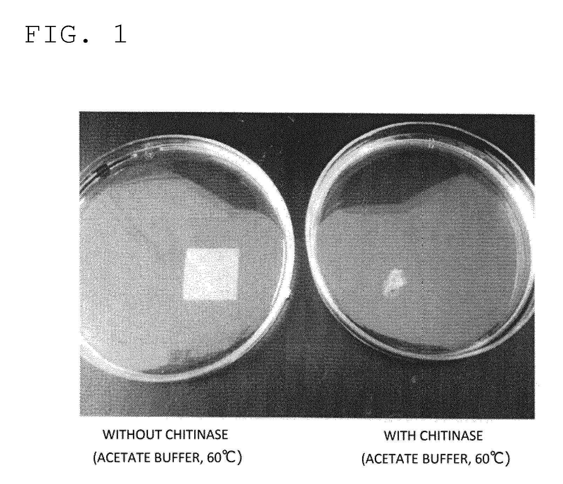

FIG. 1 is a photograph showing the result when disintegration was evaluated in a sheet comprising chitin at a temperature of 60°C in acetate buffer solution.

### Description of Embodiments

The degradable composite material of the present invention is characterized in that a degrading enzyme that can hydrolyze at least one type of polysaccharide selected from the group consisting of chitin and chitosan is supported on a molded article containing the polysaccharide (hereinafter, sometimes simply expressed as "chitinous molded article"). The best mode for carrying out the invention is described in detail below.

In general, chitin is a polysaccharide wherein N-acetylglucosamine structural units are joined by β-1,4 glycoside linkages, but in the chitin used in the present invention less than 50% of the acetyl groups can be deacetylated. In addition, chitosan is a deacetylated polysaccharide, and the chitosan used in the present invention can include parts that have not been deacetylated provided 50% or more of the acetyl groups of chitin have been deacetylated.

The chitinous molded article used in the degradable composite material of the present invention can comprise either chitin or chitosan as the polysaccharide, or it can comprise a combination thereof.

In addition, the chitinous molded article used in the degradable composite material of the present invention can be constituted of only the above polysaccharides or, as needed, can also comprise other degradable resins, additives, and the like.

The content ratio of the above polysaccharide is not particularly limited in the chitinous molded article using the degradable composite material of the present invention, but from the perspective of increasing the self-degrading capability of the degradable composite material, the total weight of polysaccharide per total weight of chitinous molded article is preferably 30 wt% to 100 wt%, more preferably 50 wt% to 100 wt%, and even more preferably 70 wt% to 100 wt%.

The configuration of the chitinous molded article of the degradable composite material of the present invention can be suitably established according to the intended mode of the degradable composite material, and is not particularly limited herein. Examples include a sheet-like, fiber (thread)-like, nonwoven fabric-like, cotton-like, sponge-like, etc. Among these sheet-like and fiber-like forms are preferred because they are configurations that are easy to use as medical materials, etc.

The chitinous molded article can be produced by publicly known manufacturing methods or can be obtained as a commercial product. The degrading enzyme used in the present invention is a thermostable enzyme. The use of such a thermostable enzyme not only can impart self-degrading capability to the degradable composite material of the present invention under high temperature conditions, but also can increase storage stability thereof. An example of such a thermostable enzyme is one with an optimal temperature of 60°C or higher, and more preferably about 90°C. The thermostable enzyme used in the present invention is a thermostable chitinase that exhibits activity at 4°C or higher and has an optimal temperature of 95°C or higher. It is one originating in *Pyrococcus furiosus.* A thermostable chitinase originating in *Pyrococcus furiosus* is already publicly known, and can be produced by the method disclosed in Japanese Patent Application Laid-open No. 2004-344160.

A preferred mode of the degradable composite material of the present invention is a chitinase supported on a molded article containing chitin; a more preferred mode is a chitinase supported on sheet-like or fiber-like molded article containing chitin; and a particularly preferred mode is a thermostable chitinase supported on a sheet-like or fiber-like molded article containing chitin.

The degrading enzyme in the degradable composite material of the present invention can be supported on the molded article containing a polysaccharide by physical adsorption or by chemical bonding. The degradable composite material of the present invention can be manufactured, for example, by applying a solution containing the degrading enzyme to the molded article containing the polysaccharide or impregnating the molded article with that solution. In addition, it can be manufactured by kneading the degrading enzyme into the molded article containing the polysaccharide.

The amount of degrading enzyme that is supported on the molded article containing the polysaccharide in the degradable composite material of the present invention is not particularly limited herein, and can be suitably established in accordance with the type of polysaccharide, mode of degradable composite material, use thereof, and the like. An example of the supported amount of degrading enzyme is 100 U to 100,000 U of degrading enzyme per 1 g of polysaccharide, preferably 1,000 U to 10,000 U, and more preferably 2,000 U to 5,000 U. For the unit of activity of the degrading enzyme used herein, 1 U is defined as the amount of enzymatic activity needed to release 1 µ mol of N-acetylglucosamine or glucosamine in 1 minute under the conditions noted below. Substrate solution: Chitin powder or chitosan powder is added to 200 mM acetate buffer (pH 5.6) to make 0.5 wt% and then suspended.
Degrading enzyme solution: The degrading enzyme to be measured is dissolved to a concentration of 0.8 mg/mL in distilled water.

### Measurement protocol:

(1) First 1 mL of substrate solution is placed in a test tube and incubated 5 min to 10 min at 37°C.
(2) Next 0.2 mL of degrading enzyme solution is added to the test tube, and the degrading enzyme is incubated under gentle agitation for 1 hour at 37°C.
(3) After the mixture has cooled, 1000 µL of DMAB (para-dimethylamino benzaldehyde) reagent is added, and after heating for 20 min at 37°C, the optical absorption is measured at 585 nm to determine the increase in reducing ends, and the released amount of N-acetylglucosamine or glucosamine is quantitated thereby. The DMAB reagent is prepared by adding 10 g of DMAB to 100 mL of acetic acid containing 12.5 wt% of 10 N hydrochloric acid, and diluting 10-fold with acetic acid immediately before use.

The uses of the degradable composite material of the present invention are not particularly limited herein, and it can be used in a variety of fields. A preferred example of the use of the degradable composite material of the present invention is a wound dressing material, medical prosthesis material, or other medical material. When the degradable composite material of the present invention is applied to the affected area, the degrading enzyme supported thereon is activated by coming into contact with the moisture present at the affected site, and self-degradation begins. Thus, because the degradable composite material of the present invention self-degrades and is absorbed *in vivo,* it is useful as a medical material (such as a wound dressing or medical prosthesis material) that will remain in a body tissue for a long period of time. In this case, when the degradable composite material of the present invention is used as a medical material, it can be applied to medical uses in humans or to medical uses in mammalian animals (nonhuman).

If the degradable composite material of the present invention is to be used in the form of a sheet, for example, it can be quite suitably used as a wound dressing material. If the degradable composite material of the present invention is to be used as a wound dressing material, the wounded area only needs to be covered with the wound dressing material. A wound dressing material that is applied to a wounded area is advantageous because it will degrade and be absorbed *in vivo* at a stage wherein the wound has healed to a certain extent, and removal after the wound has healed is unnecessary. In addition, if the degradable composite material of the present invention is used as a wound dressing material, the therapeutic effect on the wound can be increased by the gradual degradation thereof during the process of wound healing.

Moreover, if the degradable composite material of the present invention is to be used in the form of a fiber, for example, it can be quite suitably used as a medical prosthetic material. If the degradable composite material of the present invention is to be used as a medical prosthetic material, an incised or lacerated area of the patient only needs to be sutured with the medical prosthetic material. The burden on the patient can be reduced because the medical prosthetic material used for suturing an incised or lacerated area is absorbed in the body as the sutured area heals, and removal of the stitches after surgery is unnecessary. Examples

The present invention is described in detail below through examples, but is by no means limited thereto.

### Example 1

### 1. Preparation of chitinase

Agitation culturing of *E. coli* carrying a plasmid incorporating DNA that codes for a chitinase originating in *Pyrococcus furiosus* (the amino acid sequence represented by SEQ ID NO: 2 of Japanese Patent Application Laid-open No. 2004-344160) was performed at 37°C using LB culture medium (containing 10 g/L polypeptone, 5 g/L yeast extract, and 10 g/L sodium chloride), and isopropyl β-thiogalactopyranoside (IPTG) was added during the logarithmic growth phase (optical density of 0.2 to 0.4 at 600 nm) to make a final concentration of 0.2 mM. Culturing was continued unchanged overnight, and then the *E. coli* cells were collected by centrifugal separation (7 min at 6,000xg).

The *E. coli* cells recovered from 1 liter of culture liquid were suspended in 20 mL of buffer A (25 mM Tris-HCl [pH 7.5], 1 mM EDTA, 25 mM NaCl), and lysed by sonication. After lysing, a liquid extract was obtained by high-speed centrifugation (15 min at 13,000xg). The liquid extract was heated at 85°C for 30 min, high-speed centrifugation (15 min at 13,000xg) was performed once more, and the supernatant was collected.

The collected supernatant was added to a HiTrapQ anion exchange column (Amersham, 5 mL). AKTA prime (Amersham) was used for addition of the supernatant to the column and for elution. Elution was performed over a concentration gradient (25 mM to 1 M) of the salt (NaCl) contained in buffer A. The fractions wherein the target chitinase eluted were collected, and the chitinase was precipitated overnight at 4°C in 80% saturated ammonium sulfate. The precipitate was recovered by high-speed centrifugation (15 min at 13,000xg) and redissolved in buffer A. The dissolved sample was added to a HiLoad 26/60 Superdex-200pg gel filtration column (Amersham) that had been equilibrated with buffer A to remove the low molecular weight proteins and residual ammonium sulfate. The chitinase elution fractions were collected and concentrated with CentriPrep YM-10 (Amicon).

The chitinase obtained in this manner had chitinase activity of 18,000 U/g (dry weight equivalent) at 37°C.

### 2. Evaluation of degrading capability of chitinase on a sheet comprising chitin

A sheet comprising chitin (Beschitin W, Unitika) was cut into 20 mm x 20 mm squares (approximately 0.0125 g), and the squares were immersed in 20 mL of either 50 mM acetate buffer (pH 4.5) or 50 mM Tris buffer (pH 7.4). Then 0.0006 g of chitinase obtained in the above manner was added to the two solutions, and the solutions were let stand while holding the temperature at either 37°C or 60°C. After a fixed period of time, the sheets were removed and the amount of disintegration was checked. For comparison purposes, the same test was performed without adding chitinase.

FIG. 1 shows the results of the evaluation of sheet disintegration in acetate buffer at a temperature of 60°C. As shown in FIG. 1, it is clear that the sheet comprising chitin is degraded by the co-presence of chitinase. Similar results were obtained for acetate buffer at 37°C, Tris buffer at 37°C, and Tris buffer at 60°C. The sheet comprising chitin was degraded in the presence of chitinase at 37°C, and therefore the above results confirm that a chitinous sheet whereon chitinase is supported will self-degrade *in vivo* and is useful in medical applications.

### 3. Manufacture of degradable composite material

A sheet comprising chitin (Beschitin W, Unitika, 100 mm x 120 mm, 0.4 g) was immersed at 4°C for 30 min in 10 mL of 50 mM Tris buffer (pH 7.5) containing the chitinase obtained in the above manner at a concentration of 0.011 g/mL. Then, a chitin sheet whereon chitinase is supported (degradable composite material) was obtained by removing the sheet and drying it at 50°C.

## Claims

1. A degradable composite material, comprising a thermostable chitinase that exhibits activity at 4°C or higher and has an optimal temperature of 95°C or higher and is supported on a molded article containing at least one type of polysaccharide selected from the group consisting of chitin and chitosan and the molded article is a medical material that is in the form of a sheet or a fiber; wherein the thermostable chitinase is a thermostable chitinase originating in *Pyrococcus furiosus.*

2. Use of the degradable composite material according to claim 1 in the manufacture of a medical material.

3. The use according to claim 2, wherein the medical material is a wound dressing or medical prosthetic material.

4. A degradable composite material according to claim 1 wherein the molded article is in the form of a sheet, for use in a method of treating a wound by covering the wound site with the degradable composite material.

5. A degradable composite material according to claim 1 wherein the molded article is in the form of a fiber, for use in a method of suturing an incision site or a laceration site by suturing the incision site or laceration site with the degradable composite material.

## Patentansprüche

1. Abbaubares Verbundmaterial, das eine wärmebeständige Chitinase umfasst, die bei 4 °C oder mehr Aktivität zeigt und eine optimale Temperatur von 95 °C oder höher aufweist und auf einem Formteil getragen wird, das zumindest eine Art von Polysaccharid enthält, das aus der aus Chitin und Chitosan bestehenden Gruppe ausgewählt ist, wobei das Formteil ein medizinisches Material ist, das in Form einer Folie oder einer Faser vorliegt; worin die wärmebeständige Chitinase eine von *Pyrococcus furiosus* stammende wärmebeständige Chitinase ist.

2. Verwendung eines abbaubaren Verbundmaterials nach Anspruch 1 zur Herstellung eines medizinischen Materials.

3. Verwendung nach Anspruch 2, worin das medizinische Material eine Wundauflage oder ein medizinisches Prothesenmaterial ist.

4. Abbaubares Verbundmaterial nach Anspruch 1, worin das Formteil in Form einer Folie vorliegt, zur Verwendung in einem Verfahren zur Behandlung einer Wunde durch Abdecken der Wundstelle mit dem abbaubaren Verbundmaterial.

5. Abbaubares Verbundmaterial nach Anspruch 1, worin das Formteil in Form einer Faser vorliegt, zur Verwendung in einem Verfahren zum Vernähen einer Schnittstelle oder einer Rissstelle durch Vernähen der Schnittstelle oder Rissstelle mit dem abbaubaren Verbundmaterial.

## Revendications

1. Matériau composite dégradable, comprenant une chitinase termostable qui présente une activité à 4°C ou plus, et qui a une température optimale de 95°C ou plus, et qui est supportée sur un article moulé contenant au moins un type de polysaccharide sélectionné dans le groupe comprenant de chitine et de chitosane, et l'article moulé est un matériel médical qui a la forme d'une feuille ou d'une fibre ; dans lequel la chitinase termostable est une chitinase termostable ayant pour origine *Pyrococcus furiosus.*

2. Utilisation du matériau composite dégradable selon la revendication 1 dans la fabrication d'un matériel médical.

3. Utilisation selon la revendication 2, dans laquelle le matériel médical est un pansement ou un matériel prothétique médical.

4. Matériau composite dégradable selon la revendication 1, dans lequel l'article moulé a la forme d'une feuille, pour une utilisation dans un procédé de traitement d'une plaie en recouvrant le site de plaie à l'aide du matériau composite dégradable.

5. Matériau composite dégradable selon la revendication 1, dans lequel l'article moulé a la forme d'une fibre, pour une utilisation dans un procédé de suture d'un site d'incision ou d'un site de lacération en suturant le site d'incision ou le site de lacération à l'aide du matériau composite dégradable.
